(19) Europäisches Patentamt
European Patent Office
Office européen des brevets

(11) Veröffentlichungsnummer : **0 058 875**
**B1**

(12) # EUROPÄISCHE PATENTSCHRIFT

(45) Veröffentlichungstag der Patentschrift : **17.10.84**

(51) Int. Cl.³ : **C 07 C103/127**, C 07 C102/06

(21) Anmeldenummer : **82100936.2**

(22) Anmeldetag : **09.02.82**

(54) **Verfahren zur kontinuierlichen Herstellung von Formamid.**

(30) Priorität : **19.02.81 DE 3106054**

(43) Veröffentlichungstag der Anmeldung : **01.09.82 Patentblatt 82/35**

(45) Bekanntmachung des Hinweises auf die Patenterteilung : **17.10.84 Patentblatt 84/42**

(84) Benannte Vertragsstaaten : **BE DE FR GB IT NL**

(56) Entgegenhaltungen :
**DE-B- 2 623 173**
**US-A- 2 092 723**

(73) Patentinhaber : **BASF Aktiengesellschaft**
**Carl-Bosch-Strasse 38**
**D-6700 Ludwigshafen (DE)**

(72) Erfinder : **Bott, Kaspar, Dr.**
**Rieslingweg 4**
**D-6706 Wachenheim (DE)**
Erfinder : **Kaibel, Gerd**
**Robert-Bosch-Strasse 4**
**D-6840 Lampertheim (DE)**
Erfinder : **Hoffmann, Herwig, Dr.**
**Knietschstrasse 21**
**D-6710 Frankenthal (DE)**
Erfinder : **Irnich, Rudolf, Dr.**
**In den Hahndornen 2**
**D-6719 Bobenheim (DE)**
Erfinder : **Kratzer, Otto, Dr.**
**An der Tuchbleiche 7**
**D-6712 Bobenheim-Roxheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 058 875 B1

**Beschreibung**

Die vorliegende Erfindung betrifft ein verbessertes Verfahren zur kontinuierlichen Herstellung von Formamid durch Umsetzung von Methylformiat mit Ammoniak:

$$H \cdot CO{-}OCH_3 + NH_3 \longrightarrow H \cdot CO{-}NH_2 + CH_3OH$$

Diese Umsetzung ist, sieht man von der erfindungsgemäßen Verbesserung ab, allgemein bekannt, begegnet in der Praxis jedoch insofern erheblichen Schwierigkeiten, als es bisher nicht gelungen ist, das Methanol auf wirtschaftliche Weise in so reiner Form zurückzugewinnen, daß es ohne weiteres wieder in die Synthese des Methylformiates zurückgeführt werden könnte (vgl. z. B. Ullmanns Encyklopädie der technischen Chemie, 4. Auflage (1976), Band 11, S. 704).

Nach dem Verfahren der DE-PS 1 215 130 wird das Problem der Rückgewinnung von reinem Methanol dadurch gelöst, daß man bis auf das Formamid alle Komponenten des Aminolysegemisches verdampft und das Methanol aus dem Dampf fraktioniert verflüssigt. Diese Arbeitsweise ist jedoch verfahrenstechnisch umständlich und befriedigt auch hinsichtlich der Reinheit des Methanols nicht, da das Methanol immer noch etwa 500 bis 800 ppm gebundenen Stickstoff enthält.

Der Erfindung lag daher die Aufgabe zugrunde, die Herstellung von Formamid aus Methylformiat und Ammoniak verfahrenstechnisch und wirtschaftlich zu verbessern.

Demgemäß wurde gefunden, daß man Formamid aus Methylformiat und Ammoniak unter Rückgewinnung von reinem Methanol auf verfahrenstechnisch elegante Weise erhält, wenn man

a) die Umsetzung von Methylformiat und Ammoniak im oberen Bereich einer Reaktionskolonne R vornimmt, die insgesamt 15-30 theoretische Böden hat und in der bei Normaldruck ein Temperaturgradient von 110-130 °C im Sumpf bis 30-62 °C am Kopf bzw. bei leicht erhöhtem Druck ein entsprechender Temperaturgradient herrscht,

b) das bei dieser Reaktion gebildete Methanol zusammen mit geringen Mengen Formamid auf Höhe des 1. bis 5. Bodens (von unten gezählt) der Kolonne oder, falls diese mit einem zweistufigen Verdampfer ausgestattet ist, dem Verdampfer dampfförmig entnimmt,

c) von diesem Methanol/Formamidgemisch das Formamid zusammen mit geringen Mengen Methanol in einer Destillationskolonne D1 abtrennt und nach R zurückführt und

d) von dem im wesentlichen aus Formamid bestehenden Sumpfprodukt von R die darin enthaltenen geringen Mengen Methanol in einer Destillationskolonne D2 abtrennt.

Figur 1 veranschaulicht dieses Verfahren und Figur 2 betrifft eine bevorzugte verfahrenstechnische Ausgestaltung, derzufolge Methylformiat und Ammoniak vor Eintritt in die Kolonne R in einer Mischkammer M vermischt werden, in welche die aus R entweichenden Dämpfe, gegebenenfalls unter Zwischenschaltung eines (in die Figur nicht eingezeichneten) Kompressors, wieder zurückgeführt werden können.

Das Herzstück des erfindungsgemäßen Verfahrens ist die Reaktionskolonne R mit dem darin aufrechterhaltenen Temperaturgradienten, der unter Normaldruck das Intervall von 30 bis 62 °C am Kopf bis 110 bis 130 °C im Sumpf umfaßt. In der Praxis arbeitet man in dieser Kolonne vorzugsweise unter einem leicht erhöhten Druck — etwa bis zu 2 bar — weil dadurch die Kühlung am Kopf der Kolonne mit normalem Kühlwasser ermöglicht wird. Beispielsweise beträgt die Temperatur bei 2 bar am Kopf 50 bis 80 °C.

Als Bauarten der Kolonne R werden solche bevorzugt, in denen sich auf den einzelnen Böden, zumindest im Bereich der oberen 5 Böden, längere Verweilzeiten einstellen lassen. Demgemäß kommen hier Ventilböden oder vor allem Glockenböden in allen hierfür üblichen technischen Ausgestaltungen in Betracht. Für den mittleren und unteren Bereich der Kolonne sind dagegen Füllkörperschichten vorteilhafter, da in diesem Kolonnenbereich die Rückreaktion des Formamids zu Ammoniak durch möglichst kleine Flüssigkeitsverweilzeiten unterdrückt werden soll.

Im Verfahrensschritt (a) führt man dem oberen Kolonnenteil von R Methylformiat und gasförmigen oder flüssigen Ammoniak im vorzugsweise äquimolaren Mengenverhältnis zu, wobei es zweckmäßig ist, diese beiden Komponenten in einer vorgeschalteten Mischkammer intensiv zu durchmischen. Die Vermischung kann z. B. dadurch vorgenommen werden, daß die Reaktanten über Strahldüsen in einen Behälter eingeleitet werden. Dieser Behälter dient gleichzeitig als Pumpenvorlage für die Speisung der Kolonne.

Die Temperatur im oberen Kolonnenbereich wird so eingestellt, daß der überwiegende Teil des Reaktionsgemisches in flüssiger Phase vorliegt. Die Dampfphase wird wie üblich kondensiert und vollständig wieder in die Kolonne oder die Mischkammer zurückgegeben.

Unterhalb des oberen Kolonnenbereiches verdampft das Methylformiat und gelangt somit wieder in die kühlere Flüssigphase. Auch der Ammoniak, der mit der nach unten sinkenden Flüssigkeit in gelöster Form mitgeschleppt wird, wird infolge der Temperaturerhöhung aus der Flüssigphase ausgetrieben, so daß im mittleren Kolonnenteil praktisch kein Methylformiat und kein Ammoniak mehr vorhanden ist. Der Vorteil dieser Verfahrensweise ist, daß die beiden Reaktionsprodukte Formamid und Methanol dem Reaktionsgleichgewicht laufend entzogen werden, weshalb die Umsetzung zum Formamid schnell und praktisch quantitativ erfolgt.

Die mittlere Verweilzeit der Flüssigkeit im oberen Kolonnenteil, welche sich durch die Höhe der Überlaufwehre auf den Glocken- oder Ventilböden einstellen läßt, beträgt einschließlich der Verweilzeit im Mischbehälter vorzugsweise 10 bis 60 min.

Hieraus ergibt sich, daß sich im oberen Kolonnenbereich etwa 70 bis 95 Gew.-% der gesamten Füllmenge der Kolonne befinden.

Im mittleren bis unteren Kolonnenbereich verdampft das Methanol, welches gemäß Verfahrensschritt (b) der Kolonne etwa auf Höhe des 1. bis 5. Bodens dampfförmig entnommen wird. Dieses Methanol enthält, entsprechend dem Dampfdruck des Formamids, noch ungefähr 0,3 bis 1 Gew.-% Formamid.

Die Entnahme des dampfförmigen Methanolstroms kann auch direkt aus dem Verdampfer erfolgen, wenn der Verdampfer zweistufig ausgeführt wird. Dabei wird in der ersten Verdampferstufe dem aus der Kolonne kommenden methanolreichen Flüssigkeitsgemisch nur soviel Wärme zugeführt, wie man für die dampfförmig zu entfernende Methanolmenge benötigt. Diese Wärmemenge beträgt etwa zwei Drittel der gesamten Wärmemenge, wobei die Verdampfertemperatur bei Normaldruck bei etwa 95-98 °C liegt. Die restliche Wärmemenge wird in der 2. Verdampferstufe bei einer Temperatur von 125 bis 128 °C zugeführt. Aus der 1. Verdampferstufe wird der Dampf, der weniger als 1 Gew.-% Formamid enthält, abgezogen, während der Dampf der 2. Verdampferstufe mit ca. 3-5 Gew.-% Formamid in die Kolonne eingeleitet wird. Durch diese Maßnahme läßt sich ähnlich wie bei einer Seitenentnahme aus dem unteren Kolonnenbereich sicherstellen, daß der entnommene Methanoldampf nur wenig Formamid enthält.

Im Verfahrensschritt (c) wird das formamidhaltige Methanol in einer Kolonne D1 in eine Kopffraktion aus reinem Methanol und eine Sumpffraktion aus den geringen Mengen Formamid und geringen Mengen Methanol zerlegt, wonach die Sumpffraktion wieder nach R zurückgeführt wird, und zwar vorzugsweise etwas unterhalb des Methanolseitenabzuges.

Die Trennoperation in D1 ist unproblematisch, weshalb man hier Füllkörperkolonnen mit 1 bis 5 Böden bevorzugt. Anstelle einer Kolonne kann man auch ein einfaches Abscheidegefäß zur Partialkondensation des Formamides verwenden.

Das Sumpfprodukt der Reaktionskolonne R besteht im wesentlichen aus Formamid und enthält daneben noch etwa 7 bis 15 Gew.-% Methanol. Dieses Sumpfprodukt wird im Verfahrensschritt (d) in einer Kolonne D2 bei einem Druck von ca. 30 mbar in eine Sumpfaktion aus reinem Formamid und eine Kopffraktion aus Methanol zerlegt. Auch diese Destillation ist unproblematisch, so daß man hier vorzugsweise eine Füllkörperkolonne mit 5 bis 20 theoretischen Böden verwendet.

Der Stickstoffgehalt des über die Kolonnen D1 und D2 gewonnenen Methanols liegt unter 100 ppm. Dieser Wert ist bemerkenswert niedrig, da Methanol/Formamid-Gemische zur Rückspaltung in Methylformiat und Ammoniak neigen, so daß das nach den bisherigen Verfahren gewonnene Methanol stets 300 bis 500 ppm gebundenen Stickstoff enthielt und einer aufwendigen Reinigung unterworfen werden mußte.

Aufgrund eigener Untersuchungen wurde festgestellt, daß die Gleichgewichtseinstellung der eingangs formulierten Formamid-Reaktion durch die Anwesenheit von Ammoniak beschleunigt wird. Demgemäß verläuft die Rückbildung von Methylformiat und Ammoniak aus Methanol und Formamid nur sehr langsam, wenn kein Ammoniak zugegen ist. Da der Ammoniak beim vorliegenden Verfahren fast vollständig im oberen und mittleren Bereich der Reaktionskolonne zurückgehalten wird, kann es zu einer solchen Rückbildung nicht kommen, worin das hervorragende Ergebnis des erfindungsgemäßen Verfahrens, sieht man einmal von den verfahrenstechnischen Vorteilen ab, seine Erklärung findet.

Hieraus ergibt sich die verfahrenstechnisch leicht einzuhaltende Forderung, die Sumpftemperaturen in R möglichst weder zu überschreiten noch zu unterschreiten, denn bei höheren Temperaturen kann Ammoniak durch Zersetzung des Formamides freigesetzt werden und bei tieferen kann Ammoniak vom oberen Kolonnenbereich in den unteren gelangen.

Das Methanol kann man ohne weitere Behandlung wieder in die Stufe der Methylformiat-Synthese zurückführen. Da bei dem Verfahren praktisch kein Methanol verloren geht, bildet das Methanol über die Synthesestufe und die Methylformiat-Aminolyse einen geschlossenen Kreislauf.

Im Hinblick auf Energieeinsparungen kann das erfindungsgemäße Verfahren noch ausgestaltet werden, indem man vor dem Kondensator eine Dampfverdichtung auf 10 bis 15 bar vornimmt. Die Kondensationstemperatur steigt hierdurch auf bis zu 150 °C an. Bei dieser hohen Temperatur findet in der Pumpenvorlage für den Kolonnenrücklauf, die gleichzeitig als Mischbehälter dient, eine rasche Reaktion statt. Wenn die Kolonne bei Normaldruck betrieben wird, ist es möglich, die Kopfwärme zum Betreiben der Verdampfer am Kolonnensumpf zu benutzen und damit einen entsprechenden Betrag an Heizdampf einzusparen. Der Ammoniak kann flüssig oder dampfförmig zugegeben werden. Bei der Arbeitsweise mit erhöhtem Kondensatordruck ist die Verwendung flüssigen Ammoniaks zu bevorzugen.

Das Formamid fällt in praktisch quantitativer Ausbeute und in einer so hohen Reinheit an, daß es für die meisten Zwecke nicht weiter gereinigt zu werden braucht.

## Beispiel

In den oberen Bereich einer bei Normaldruck betriebenen 2,5 m hohen Reaktions-Glockenbodenkolonne R, die 10 theoretische Böden hatte und auf eine Füllkörperkolonne mit 15 theoretischen Böden

aufgesetzt war, wurden stündlich 1 100 g Methylformiat und 312 g Ammoniak (Molverhältnis 1 : 1) gegeben.

In den oberen 10 Böden herrschte eine Temperatur von 59 bis 74 °C, unter der alle Reaktionsteilnehmer in überwiegend flüssiger Phase vorlagen.

Die mittlere Verweilzeit der Reaktionsteilnehmer in diesem Bereich betrug etwa 50 min, entsprechend einem Anteil von etwa 80 Gew.-% der Kolonnenfüllung im oberen Bereich. Die Kolonne wurde unter vollständigem Rücklauf betrieben.

Auf Höhe des 1. Bodens (von unten gezählt) wurde der Kolonne bei 98 °C stündlich ein dampfförmiges Gemisch aus 517 g Methanol und 3 g Formamid entnommen. Dieses Gemisch wurde in einer kurzen Füllkörperkolonne mit 8 theoretischen Böden in eine Kopffraktion von 505 g/h Methanol und eine Sumpffraktion aus 12 g/h Methanol und 3 g/h Formamid zerlegt. Die Sumpffraktion wurde auf Höhe des 1. Bodens wieder in die Kolonne R zurückgeführt. Vom Sumpf der Kolonne R wurde bei 128 °C stündlich ein flüssiges Gemisch aus 825 g Formamid und 82 g Methanol abgezogen, welches in einer Füllkörperkolonne mit 15 theoretischen Böden in eine Kopffraktion aus 82 g/h Methanol und 21 g/h Formamid sowie eine Sumpffraktion aus 804 g/h Formamid zerlegt wurde. Die Reinheit des Formamids betrug > 99 % und das Methanol enthielt weniger als 100 ppm Stickstoff.

**Ansprüche**

1. Verfahren zur kontinuierlichen Herstellung von Formamid aus Methylformiat und Ammoniak unter Rückgewinnung von reinem Methanol, dadurch gekennzeichnet, daß man

a) die Umsetzung von Methylformiat und Ammoniak im oberen Bereich einer Reaktionskolonne R vornimmt, die insgesamt 15-30 theoretische Böden hat, und in der bei Normaldruck ein Temperaturgradient von 110-130 °C im Sumpf bis 30-62 °C am Kopf bzw. bei leicht erhöhtem Druck ein entsprechender Temperaturgradient herrscht,

b) das bei dieser Reaktion gebildete Methanol zusammen mit geringen Mengen Formamid auf Höhe des 1. bis 5. Bodens (von unten gezählt) der Kolonne oder, falls diese mit einem zweistufigen Verdampfer ausgestattet ist, dem Verdampfer dampfförmig entnimmt,

c) von diesem Methanol/Formamidgemisch das Formamid zusammen mit geringen Mengen Methanol in einer Destillationskolonne D1 abtrennt und nach R zurückführt und

d) von dem im wesentlichen aus Formamid bestehenden Sumpfprodukt von R die darin enthaltenen geringen Mengen Methanol in einer Destillationskolonne D2 abtrennt.

2. Verfahren nach Anspruch 1, dadurch gekennzeichnet, daß man das Methylformiat und den Ammoniak vor Eintritt in die Kolonne R in einer Mischkammer M vermischt.

**Claims**

1. A process for the continuous preparation of formamide from methyl formate and ammonia, pure methanol being recovered, wherein

a) the reaction of methyl formate and ammonia is carried out in the upper region of a reaction column R which has a total of from 15 to 30 theoretical plates and in which, at atmospheric pressure, a temperature gradient ranging from 110-130 °C at the bottom to 30-62 °C at the top prevails or, at slightly elevated pressure, a corresponding temperature gradient prevails,

b) the methanol formed in this reaction is removed, together with small amounts of formamide, from the column at the level of plates 1-5 (counted from the bottom) or, if the column is provided with a two-stage evaporator, is removed from the evaporator in vaporous form,

c) the formamide is removed from this methanol/formamide mixture, together with small amounts of methanol, in a distillation column D1 and recycled to R, and

d) the small amounts of methanol contained in the bottom product from R, which consists substantially of formamide, are separated off in a distillation column D2.

2. A process as claimed in claim 1, wherein the methyl formate and the ammonia are mixed in a mixing chamber M before entering column R.

**Revendications**

1. Procédé de préparation continue de formamide à partir de formiate de méthyle et d'ammoniac avec récupération d'alcool méthylique pur, caractérisé en ce que :

a) la réaction du formiate de méthyle et de l'ammoniac est réalisée dans la partie supérieure d'une colonne de réaction R, comportant au total entre 15 et 30 plateaux théoriques et dans laquelle est maintenu un gradient de températures entre 110 et 130 °C à la base et 30 à 62 °C au sommet, à la pression normale, ou un gradient aaproprié dans le cas d'une pression légèrement accrue ;

b) l'alcool méthylique formé est soutiré, avec une proportion mineure de formamide, au niveau du

1er plateau ou entre le 1er et le 5e plateau (comptés du bas) de la colonne ou de l'évaporateur, si la colonne est équipée d'un évaporateur à deux étages, à l'état gazeux ;

c) du mélange alcool méthylique-formamide, on sépare dans une colonne de distillation D1 le formamide et une proportion mineure d'alcool méthylique, que l'on recycle dans la colonne R ;

d) du produit résiduel dans la colonne R, composé essentiellement de formamide, on élimine les proportions mineures d'alcool méthylique dans une colonne de distillation D2.

2. Procédé suivant la revendication 1, caractérisé en ce que le formiate de méthyle et l'ammoniac sont mélangés dans une chambre de mélange M avant leur introduction dans la colonne R.

FIG.1

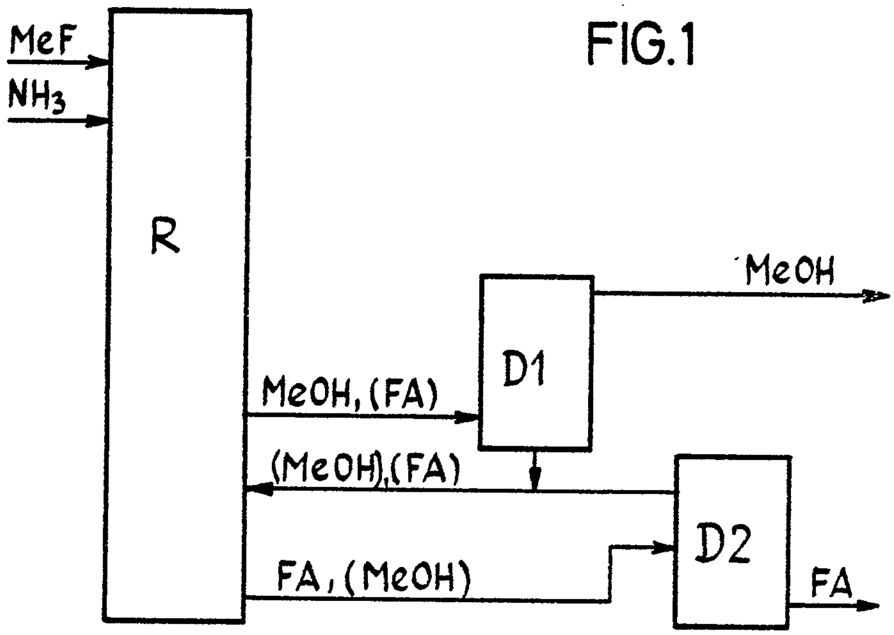


MeF = Methylformiat
MeOH = Methanol
FA = Formamid
( ) = geringe Mengen

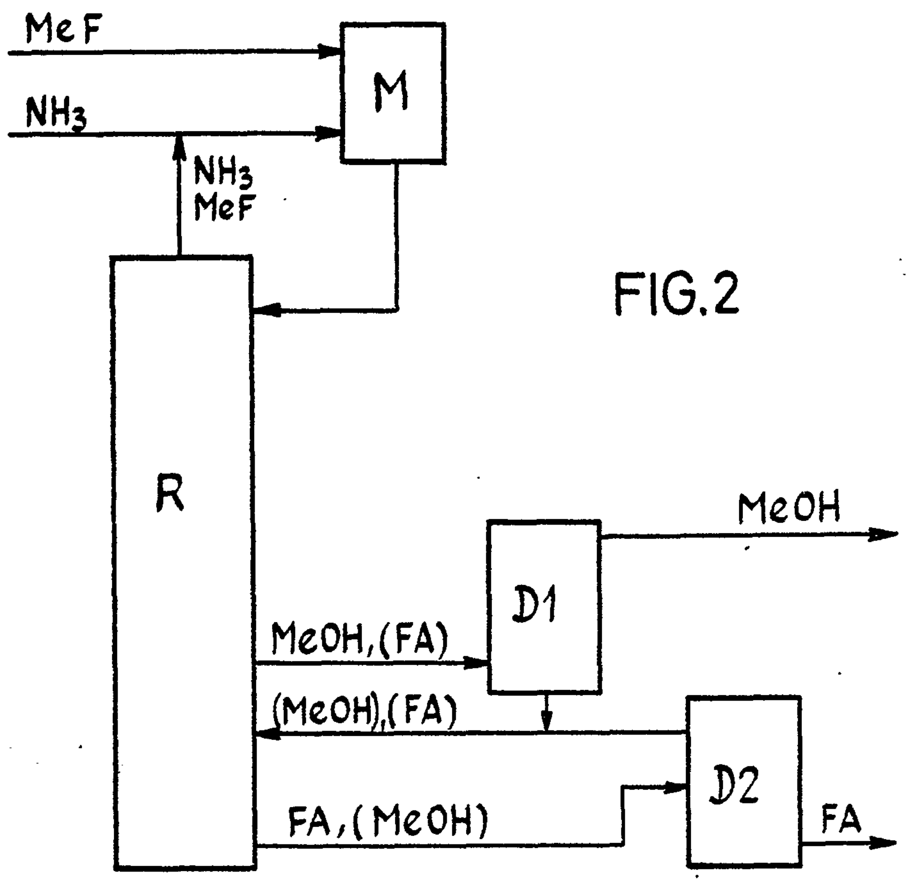


FIG.2

Legende s. Fig.1